# EUROPÄISCHE PATENTSCHRIFT

(11) **EP 2 512 400 B1**
(45) Veröffentlichungstag und Bekanntmachung des Hinweises auf die Patenterteilung: **25.09.2013**
(21) Anmeldenummer: 10798538.4
(22) Anmeldetag: 16.12.2010
(51) Int. Cl.: A61K 6/00, A61K 6/083

(54) **Mehrkomponentensystem zur Herstellung eines Dentalmaterials**
Multicomponent system for producing a dental material
Système à plusieurs composants pour la fabrication d'un matériau dentaire

(30) Priorität: 16.12.2009 DE 102009058638
(43) Veröffentlichungstag der Anmeldung: 24.10.2012
(73) Patentinhaber: Ernst Mühlbauer GmbH & Co.KG, 25870 Norderfriedrichskoog (DE)
(72) Erfinder: NEFFGEN, Stephan, 25421 Pinneberg (DE); HAUSER, Karsten, 22529 Hamburg (DE)
(74) Vertreter: Glawe, Delfs, Moll
(86) Internationale Anmeldenummer: PCT/EP2010/069975
(87) Internationale Veröffentlichungsnummer: WO 2011/083020

(56) Entgegenhaltungen:
- DE-A1- 19 719 890
- DE-A1- 19 928 238
- US-A- 4 145 378
- US-A- 5 688 883
- US-B1- 6 437 065

## Beschreibung

Die Erfindung betrifft ein Zwei- oder Mehrkomponentensystem zur Herstellung eines Dentalmaterials gemäß dem Oberbegriff des Anspruchs 1, einen Kit mit diesem System sowie die Verwendung des Systems zur Herstellung eines Dentalmaterials.

Polymerisierbare Dentalmaterialien der eingangs genannten Art sind beispielsweise aus WO 00/78271 A1 bekannt und finden Verwendung beispielsweise als Füllungsmaterialien, Adhäsive, Stumpfaufbaumaterialien, Kronen- und Brückenmaterialien sowie provisorischen Kronen- und Brückenmaterialien, und Zemente.

Zur Herstellung von Dentalmaterialien werden die Komponenten des Systems zu einer pastösen Masse vermischt und appliziert. Diese Masse wird durch radikalische Polymerisation ausgehärtet.

Verschiedene Initiatorsysteme für die radikalische Polymerisation sind im Stand der Technik beschrieben.

Beispielsweise aus EP 0 374 824 A1 sind Amin-Peroxid-Systeme zur Polymerisationsinitiation bekannt. Die eingesetzten Amine können toxisch bedenklich sein, ferner führen sie häufig zu ästhetisch nicht akzeptablen Verfärbungen des Restaurationsmaterials. Unter Umständen kann es zudem zu verhältnismäßig hohen Temperaturen bei der Aushärtung kommen, die angrenzendes Zahnmaterial wie beispielsweise die Pulpa schädigen können.

Aus DE 199 28 238 A1 ist ein Redoxinitiatorsystem bekannt, welches ein Barbitursäurederivat und/oder ein Malonylsulfamid sowie einen Carbonsäureperoxyester umfasst.

Der Erfindung liegt die Aufgabe zugrunde, ein System der eingangs genannten Art zu schaffen, das in der Praxis mit guten Ergebnissen verarbeitbar und aushärtbar ist und eine gute Lagerstabilität aufweist.

Die Erfindung löst die Aufgabe bei eingangs genannten Systemen dadurch, dass die erste und/oder zweite Komponente mindestens ein Perketal enthält.

Zunächst seien einige im Rahmen der Erfindung verwendete Begriffe erläutert.

Gegenstand der Erfindung ist ein Zwei- oder Mehrkomponentensystem, bevorzugt ein Zweikomponentensystem. Das Dentalmaterial wird somit aus wenigstens zwei, bevorzugt genau zwei Komponenten angesetzt.

Die erste Komponente enthält wenigstens ein radikalisch polymerisierbares Harz sowie einen Polymerisationsbeschleuniger, der selbst nicht als Polymerisationsinitiator wirkt und somit die Lagerstabilität der ersten Komponente nicht oder nicht wesentlich beeinträchtigt. Als Polymerisationsbeschleuniger werden vorzugsweise insbesondere Kombinationen von zweiwertigen Übergangsmetallionen und Halogenid- oder Pseudohalogenidionen, insbesondere Chloridionen, verwendet.

Die zweite Komponente enthält als Polymerisationsinitiator eine CH-acide Verbindung und/oder ein Salz einer CH-aciden Verbindung.

Entsprechende CH-acide Verbindungen wurden intensiv von H. Bredereck und seinen Mitarbeitern untersucht (H. Bredereck et al.: "Über CH-Aktive Polymerisationsinitiatoren - XIII. Mitt. Polymerisationen und Polymerisationsinitiatoren", die Makromolekulare Chemie 92 [1966] S. 70-90; H. Bredereck et al.: "Polymerisationen und Polymerisationsinitiatoren - 16. Einfluß von Thio-Gruppen in Barbitursäurederivaten auf die Polymerisationsauslösung von Methacrylsäure-methylester", die Makromolekulare Chemie 176 [1975] S. 1713-1723). Von den CH-aciden Verbindungen haben sich im Dentalbereich die Barbitursäurederivate als günstig erwiesen. Sie sind in hohen Ausbeuten und Reinheiten darstellbar, industriell verfügbar (Chemische Fabrik Berg GmbH, Mainthalstr. 3, D-06749 Bitterfeld) und erlauben durch ihre Reaktionskinetik die Realisierung interessanter Eigenschaften.

Die Synthese der Barbitursäurederivate ist z.B. aus E. Fischer und A. Dilthey ("Über c-Dialkylbarbitursäuren und über die Ureide der Dialkylessigsäuren", Ann. 335 [1904] S. 335) bekannt und beschreibt die alkalische Kondensation von Derivaten des Malonsäurediethylesters mit N-substituiertem Harnstoff in Natriumalkoholat. Die dabei erhaltenen Natriumsalze der Barbitursäurederivate werden anschließend durch die Zugabe einer Säure, z.B. von Salzsäure, in die Barbitursäurederivate überführt.

Bei dem auf Barbitursäure bzw. deren Derivaten basierenden Initiatorsystem müssen die Barbitursäurederivate von den polymerisierbaren Monomeren getrennt aufbewahrt werden. Dies liegt darin begründet, dass CH-acide Verbindungen wie die Derivate der Barbitursäure bereits ohne die Mitwirkung von Cu(II)- und Clorid-Ionen durch Autoxidation durch Luftsauerstoff Hydroperoxide bilden. Diese Hydroperoxide zerfallen unter Bildung von Radikalen, welche die Polymerisation der reaktiven Monomere initiieren, so dass es binnen kurzer Zeit zur spontanen Polymerisation kommt. Dieser spontane Polymerisationsprozess kann durch Zugabe von Stabilisatoren für kurze Zeit verzögert oder unterdrückt werden, nicht hingegen über einen längeren Zeitraum, wie es bei lagerstabilen Systemen erwünscht ist.

Bestandteil der zweiten Komponente ist deswegen in einer Variante der Erfindung eine bei Raumtemperatur flüssige oder pastöse inerte Matrix, die durch die CH-acide Verbindung nicht zur Polymerisation gebracht werden kann. Geeignet sind beispielsweise Weichmacher wie zum Beispiel Polyethylenglykole, die unten noch näher beschrieben werden.

In einer zweiten Variante der Erfindung kann die zweite Komponente ebenfalls radikalisch polymerisierbare Harze enthalten. Voraussetzung dafür ist, dass die in der zweiten Komponente enthaltene CH-acide Verbindung als Salz vorliegt. Eine als Salz vorliegende CH-acide Verbindung kann in dieser Salzform noch nicht als Polymerisationsinitiator wirken. Zu diesem Zweck muss sie zuvor beispielsweise mittels einer Säure in die entsprechende CH-acide Verbindung selbst überführt werden. Bei dieser Ausführungsform der Erfindung kann die zweite Komponente beispielsweise eine basische Verbindung enthalten, die das Salz der CH-aciden Verbindung stabilisiert und bevorzugt die Peroxidkomponente nicht zersetzt. Die erste Komponente kann eine geeignete Säure enthalten, die in der Lage ist, das Salz der CH-aciden Verbindung zu protonieren. Nach dem zusammenmischen der beiden Komponenten protoniert die Säure das Salz zu der freien CH-aciden Verbindung, die dann als Polymerisationsinitiator wirkt.

In einer weiteren bevorzugten Ausführungsform der Erfindung enthält die erste Komponente wenigstens eine radikalisch polymerisierbare Säure. Geeignete radikalisch polymerisierbare Säuren sind insbesondere die im Rahmen der Erfindung als Bestandteil der ersten Komponente verwendbaren radikalisch polymerisierbaren Harze, die Säuregruppen enthalten, beispielsweise Carbon-, Sulfon-, Phosphon- oder Phosphorsäuregruppen.

Die Erfindung hat erkannt, dass insbesondere die mechanischen Eigenschaften von Dentalmaterialien nach der Aushärtung sich gegenüber dem Stand der Technik, der CH-acide Verbindungen und Übergangsmetallionen einsetzt, wesentlich verbessern lassen durch den Einsatz von Perketalen. Überraschenderweise wird erfindungsgemäß eine hohe Lagerstabilität der noch nicht ausgehärteten Komponenten des Systems erreicht, ferner gute mechanische Eigenschaften des ausgehärteten Dentalmaterials nach längerer Lagerung der Komponenten.

Erfindungsgemäß ist das Peroxid bevorzugt in der zweiten Komponente enthalten. Der Gehalt des Zweikomponentensystems an den erfindungsgemäß zugesetzten Peroxiden beträgt bevorzugt 0,001 bis 5 Gew.-%, weiter vorzugsweise 0,01 bis 3 Gew.-%, weiter vorzugsweise 0,02 bis 2 Gew.-%, weiter vorzugsweise 0,02 bis 1,5 Gew.-%, weiter vorzugsweise 0,03 bis 1 Gew.-%. Die genannten Ober- und Untergrenzen sind beliebig zu erfindungsgemäßen Bereichen kombinierbar.

Überraschenderweise sind die Komponenten der erfindungsgemäßen Dentalmaterialien besonders lagerstabil und weisen auch nach längerer Lagerung eine gleichbleibende und gewünschte Erhärtungscharakteristik auf. Mechanische Eigenschaften wie Härte und insbesondere Biegefestigkeit/Biegemodul des ausgehärteten Dentalmaterials wird durch längere Lagerung der Komponenten vor der Verarbeitung und Aushärtung nicht oder nur unwesentlich beeinflusst.

Unter den erfindungsgemäß eingesetzten Perketalen kommen bevorzugt diejenigen zum Einsatz, deren sogenannte 10-Stunden-Halbwertstemperatur mindestens 75° C, vorzugsweise mindestens 80° C beträgt. Dies bedeutet, dass nach zehnstündiger Lagerung des Peroxids bei dieser Temperatur die Hälfte der Peroxidprobe zerfallen ist. Die Halbwertszeit ist die Zeit, in der die Hälfte der Peroxidmenge in einem bestimmten Lösungsmittel zerfällt. Die Bestimmung der Halbwertszeiten erfolgte in 0,1 molarem Monochlorbenzen.

Perketale (R¹₂C(-O-O-R²)₂) sind Verbindungen, die sich von Ketalen durch formalen Ersatz der Sauerstoff-Brücke zwischen den Alkylgruppen durch eine Sauerstoff-Sauerstoff-Brücke herleitet (Ersatz eines Sauerstoffs durch eine Peroxogruppe).

Geeignete Perketale sind aliphatische oder cyclische Perketale.

Geeignete Perketale sind beispielsweise 1,1-Di-(tert.-butylperoxy)-3,3,5-trimethylcyclohexan; 1,1-Di-(tert.butylperoxy)-cyclohexan; 2,2-Di-(tert.-butylperoxy)-butan, 1,1-Di-(tert.-amylperoxy)-cyclohexan, Butyl-4,4-di-(tert.-butylperoxy)-valerat, Ethyl-3,3-di-(tert.-amylperoxy)-butanoat und Ethyl-3,3-di-(tert.-butylperoxy)-butanoat. Ein bevorzugtes cyclisches Perketal ist beispielsweise 3,6,9-Triethyl-3,6,9-trimethyl-1,4,7-triperoxononan.

Geeignete CH-acide Verbindungen sind dem Fachmann bekannt. Geeignete CH-acide Verbindungen sind solche, die Wasserstoff an C-Atomen in α-Position zu einer, bevorzugt mehreren elektronenziehenden Gruppen aufweisen. Beispiele geeigneter CH-acider Verbindungen sind α-Benzoylpropionitrile, α-Cyancarbonsäureester und - amide, cyclische β-Oxonitrile, β-Diketone, cyclische β-Diketone, cyclische β-Oxocarbonsäureester, cyclische β-Oxolactone, Malonsäuren, insbesondere Malonylsulfamid, Pyrazole, insbesondere Pyrazolon und Pyrazolidin, Barbitursäure, Barbitursäurederivate, Thiobarbitursäure, Thiobarbitursäurederivate sowie die Salze, insbesondere Erdalkali-, Alkalisalze sowie teilweise oder vollständig alkylierte oder arylierte oder gemischt alkylierte oder arylierte Ammonium- oder Phosphoniumsalze vorgenannter Verbindungen. Auch andere organisch modifizierte Kationen sind einsetzbar. Bevorzugt sind als Barbitursäurederivate 1,3,5-Dimethylbarbitursäure, 1,3-Dimethyl-5-ethylbarbitursäure, 1,5-Dimethylbarbitursäure, 1-Methyl-5-ethylbarbitursäure, 1-Methyl-5-propylbarbitursäure, 5- Ethylbarbitursäure, 5- Propylbarbitursäure, 5-Butylbarbitursäure und 1-Cyclohexyl-5-Etylbarbitursäure. Besonders bevorzugt ist das Barbitursäurederivat 5-Phenyl-1-benzylbarbitursäure (PBS).

Ionogene Halogenid oder Pseudohalogenidverbindungen sind bevorzugt so ausgewählt, dass sie eine ausreichende Löslichkeit in der jeweiligen Komponente besitzen. Besonders bevorzugt sind hier Ammoniumsalze, insbesondere Tetraalkylammoniumsalze wie sie in EP 2 070 506 (Lück) beschrieben sind. Als Halogenide sind Chloride besonders bevorzugt.

Bevorzugte Polymerisationsbeschleuniger umfassen im Harzsystem lösliche Metallverbindungen enthaltend Metallionen, die zum Wechsel der Oxidationsstufe fähig sind, wie Cu(II), Fe(II), Fe(III) oder Co-Salze organischer Säuren oder deren Komplexe. Besonders bevorzugt sind Verbindungen des Kupfers wie Cu(II)Sulfat (wasserfrei und Hydratformen), CuCl₂, Kupferacetat, Kupferacetylacetonat, Kupfernaphthenat, Kupfersalicylat, Kupferkomplexe mit EDTA, Bis(1-Phenylpentan-1,3-dionato)kupfer, Kupferdimethacrylat und Kupferbenzoylacetonat.

In einer bevorzugten Ausführungsform befinden sich ionogene Verbindung und Polymerisationsbeschleuniger zusammen in der die polymerisierbaren Harze enthaltenden ersten Komponente.

Es kann bevorzugt sein, als weitere Komponente des Initiatorsystems zusätzliche (nicht CH-acide) Reduktionsmittel zu verwenden. Geeignete zusätzliche Reduktionsmittel sind beispielsweise in der EP 0732098 beschrieben. Ferner sind hier N,N-dialkylierte aromatische Amine wie N,N-Bishydroxyethyl-p-toluidin, oder N,N-dialkylierte Aminobenzoesäureester wie p-N,N-Dimethylaminonezoesäureethylester geeignet. Aminverbindungen sind besonders bevorzugt. Die zusätzlichen Reduktionsmittel sind bevorzugt in der ersten Komponente enthalten, die nicht das Peroxid enthält.

Das erfindungsgemäße Initiatorsystem kann ein fotochemisch aktivierbares Initiatorsystem einschließen. Bevorzugte sind im Blaulichtbereich zu aktivierende Initiatorsysteme wie 1,2-Dicarbonylverbindungen, beispielsweise Campherchinon-Amin-Systeme und/oder auch im UV-Licht-Bereich zu aktivierende beispielsweise Phosphinoxide. Die bevorzugten Fotoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, α-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethyl-benzoyl-phosphinoxid, Benzoin, Benzoinalkylether, Benzildialkylketale, α-Hydroxy-acetophenon, Dialkoxyacetophenone, α-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (eta-6-Cumol)(eta-5-cyclopentadienyl)eisen-tetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, α-Dicarbonylverbindungen, kondensierte Polyaromaten oder eine Mischung aus diesen Verbindungen eingesetzt. Besonders bevorzugt werden N,N-Dimethyl-p-toluolidin, N,N-Dialkyl-alkyl-aniline, N,N-Dihydroxyethyl-p-toluidin, 2-Ethylhexyl-p-(dimethyl-amino)-benzoat, Butyrylcholin-triphenylbutyl-borat oder eine Mischung aus diesen Verbindungen eingesetzt

Harze sind bevorzugt die dem Fachmann bekannten dentalüblichen (Meth)acrylate, Di(meth)acrylate und/oder höher funktionalisierte (Meth)acrylate. Die Allgemeinheit nicht einschränkend sind nachfolgend einige Beispiele genannt:
Methyl(meth)acrylat, Ethyl(meth)acrylat, n- oder i-Propyl(meth)acrylat, n-, i- oder tert.-Butyl(meth)acrylat, Hexyl(meth)acrylat, 2-Ethylhexyl(meth)acrylat, Cyclohexyl(meth)acrylat, Isobornyl(meth)acrylat, Hydroxyethyl(meth)acrylat, Hydroxypropyl(meth)acrylat, Phosphorsäureester von Hydroxyethyl(meth)acrylat bzw. Hydroxypropyl(meth)acrylat, (Meth)acrylsäure, Malonsäure-mono-(meth)acrylatester, Bernsteinsäure-mono-(meth)acrylatester, Maleinsäure-mono-(meth)acrylatester, Glycerin(meth)acrylat, Glycerin(meth)acrylatester, Glycerindi(meth)acrylat, Glycerin-di(meth)acrylatester (wie z.B. Glycerin-di(meth)acrylatsuccinat), 4-(Meth)acryloyloxyethyltrimellithsäure, Bis-4,6- bzw. Bis-2,5-(Meth)acryloyloxyethyltrimellithsäure, 2-(((Alkylamino)-carbonyl)oxy)ethyl-(meth)acrylate, Allyl(meth)acrylat, Tetrahydrofurfuryl(meth)acrylat, Butandioldi(meth)acrylat, Hexandiol-di(meth)acrylat, Decandioldi(meth)acrylat, Dodecandiol-di(meth)acrylat, Ethylenglykoldi(meth)acrylat, Diethylenglykol-di(meth)acrylat, Triethylenglykoldi(meth)acrylat, Tetraethy-lenglykoldi(meth)acrylat, Polyethylenglykoldi(meth)acrylate, Glycerindi(meth)acrylat, Glycerolpropoxytri(meth)acrylat, Trimethylolpropantri(meth)acrylat, ethoxylierte und/oder propoxylierte Trimethylolpropantri(meth)acrylate, Pentaerythrittri(meth)acrylat, Pentaerythrittetra(meth)acrylat, Dipentaerythritolpenta(meth)acrylat, Dipentaerythritolhexa(meth)acrylat, Bisphenol-A-di(meth)acrylat, ethoxylierte und/oder propoxylierte Bisphenol-A-di(meth)acrylate, 2,2-Bis-4(3-(meth)acryloxy-2-hydroxy-propoxy)-phenylpropan und davon abgeleitete Verbindungen, Chlor- und Bromphosphorsäureester des Bisphenol-A-glycidyl(meth)acrylats, Urethan(meth)acrylate wie z.B. 7,7,9-Trimethyl-4,13-dioxo-3,14-dioxa-5,12-diazahexadecan-1,16-dioxy-dimethacrylat, di- oder höher funktionelle oligomere oder polymere Urethan(meth)acrylate wie sie bspw. in EP 1237525 oder in EP 1242493 beschrieben sind, Polyester(meth)acrylate, Polycarbonat(meth)acrylate, Polyamid(meth)acrylate, Polyimid(meth)acrylate, Phosphazen(meth)acrylate und Siloxan(meth)acrylate.

Aber auch andere radikalisch polymeriserbare Systeme sind möglich. Die Monomere können neutral, basisch oder sauer sein. Die Monomermoleküle können verschiedenste Funktionalitäten enthalten wie Hydroxyfunktionen, Aminofunktionen, Carboxylfunktionen sowie weitere übliche organische Funktionalitäten. Ebenso bevorzugt enthalten sein können die in EP 2016931 beschriebenen Dimethacrylate mit Tricyclodecan-Kerngerüsten.

Geeignete Weichmacher sind dem Fachmann bekannt. Darunter fallen z. B. Polethylenglykole, Polypropylenglykole, ungesättigte und gesättigte Polyester, Phthalate, Adipate, Sebacate, Phosphorsäureester, Phosphonsäureester und/oder Zitronensäureester. Beispiele besonders geeigneter Weichmacher sind Polethylenglycolderivate wie Polyethylenoxid, 2,2-Bis-[4-[oligo(ethoxy)]phenyl]propan-diacetat, 2,2-Bis-[4-[2-hydroxyethoxy]phenyl]propan-diacetat, Polypropylenglycolderivate, andere Poly- und Oligoether, niedermolekulare Polyester, Phthalsäureester, Silikonöle, Paraffinöle und ähnliches.

Eine oder beide Komponenten des erfindungsgemäßen Zweikomponentensystems enthalten bevorzugt zusätzlich dentalübliche Additive, beispielsweise Additive ausgewählt aus der Gruppe bestehend aus Füllstoffen und Thixotropiermitteln.

Das erfindungsgemäße System kann in mindestens einer der Komponenten Füllstoffe enthalten. Bei den erfindungsgemäß eingesetzten Füllstoffen handelt es sich bevorzugt um nano- und/oder mikroskalige (teilweise röntgenopake) Füllstoffe, vorzugsweise um Glaspulver, Glaskeramikpulver, Metall-, Halbmetall- oder Mischmetalloxide, Silikat-, Nitrid-, Sulfat-, Titanat-, Zirkonat-, Stannat-, Wolframat-, Siliciumdioxid- Verbindungen oder eine Mischung aus diesen Verbindungen oder sphärische Füllstoffe, Quarzpulver, β-Cristobalit, röntgenopake Dentalgläser oder eine Mischung aus diesen Pulvern oder gefüllte oder ungefüllten Splitterpolymerisate und/oder Perlpolymerisate.

Bei den erfindungsgemäß eingesetzten nanoskaligen Füllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Der Füllstoff kann gemäß einer bevorzugten Ausführungsform der Erfindung ein oberflächenmodifizierter Füllstoff, vorzugsweise ein organisch oberflächenmodifizierter Füllstoff sein. Der Füllstoff kann nach seiner Oberflächenmodifikation, beispielsweise einer Silanisierung, auf seiner Oberfläche funktionelle Gruppen, beispielsweise reaktive Methacrylatgruppen besitzen, die mit den Monomeren chemisch, vorzugsweise radikalisch, reagieren können oder eine hohe Affinität zu der aus den Monomeren gebildeten Polymermatrix aufweisen.

Das erfindungsgemäße System kann zur Einstellung bestimmter Eigenschaften zusätzlich so genannte Additive bzw. Modifikatoren enthalten. Die Allgemeinheit nicht einschränkend sind nachfolgend einige Beispiele genannt:
Anorganische und/oder organische Farbpigmente bzw. Farbstoffe, Fluoreszenzfarbstoffe zur Erreichung der natürlichen Fluoreszenz des Zahnes, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester), Aromastoffe, und Duftstoffe.

Neben dem Zusatz dem Fachmann bekannter und dentalüblicher Additive kann es vorteilhaft sein, Trockenmittel zur Wasserabsorption als Bestandteile zu verwenden. Geeignete Trockenmittel sind bspw. Zeolithe, wasserfreie oder entwässerte Salze wie MgSO₄ oder CaSO₄-Halbhydrat getrocknetes Kieselgel, CaCl₂ und Silicagele. Besonders bevorzugt ist die Verwendung eines Trockenmittels in der Komponente, die das Peroxid enthält.

Die Komponenten liegen als Flüssigkeiten, bevorzugt als (fließfähige) Pasten vor. Die Herstellung geschieht durch geeignetes Vermischen der Bestandteile der einzelnen Komponenten durch geeignete Verfahren wie Rühren, Kneten, Dispergieren, Walzen u.a. bis homogene Komponenten erhalten werden.

Die Komponenten des Materials werden zur Erhärtung per Hand oder bevorzugt automatisch mittels statischer oder dynamischer Mischer (beispielsweise der Fa. Sulzer MixPac®) miteinander gemischt. Gegebenenfalls können weitere Komponenten zugemischt werden.

Die Komponenten können saure Bestandteile, wie polymerisierbare Säuren oder auch nicht polymerisierbare Säuren enthalten. Schwache, mittelstarke oder auch starke Säuren können hier geeignet sein. In einer speziellen Ausführungsform umfasst die Komponente, die nicht das Peroxid enthält, hydroxyfunktionelle Verbindungen und/oder Wasser. Eine optionale Säure, bevorzugt eine mittelstarke bis starke Säure, kann dann zusätzlich in einer der beiden oder in beiden Komponenten enthalten sein.

Das erfindungsgemäße Zwei- oder Mehrkomponentensystem kann zur Herstellung eines Dentalmaterials verwendet werden. Das hergestellte Dentalmaterial ist bevorzugt ausgewählt aus der Gruppe bestehend aus Füllungsmaterialien, Adhäsiven, Stumpfaufbaumaterialien, Kronen- und Brückenmaterialien, provisorischen Kronen- und Brückenmaterialien, sowie Zementen.

Gegenstand der Erfindung ist ferner ein Kit zur Herstellung eines Dentalmaterials, das ein erfindungsgemäßes Zwei- oder Mehrkomponentensystem in geeigneten Behältern enthält. Es kann sich um geeignete Vorratsbehälter, Kartuschen für statische oder dynamische Mischer oder dergleichen handeln. Bestandteil des Kits kann eine geeignete Mischvorrichtung wie ein bereits genannter statischer oder dynamischer Mischer sein.

Ausführungsbeispiele der Erfindung werden nachfolgend beschrieben. Die folgenden kommerziell erhältlichen Bestandteile kamen zum Einsatz:

| | |
|---|---|
| BisGMA | Bisphenol-A-diglycidyldimethacrylat (CAS 001565-94-2) |
| TEDMA | Triethylenglycoldimethacrylat |
| UDMA | 7,7,9-Trimethyl-4,13-dioxo-3,14-dioxa-5,12-diazahexadecan-1,16-dimethacrylat (CAS 72869-86-4) |
| BPA(EO)₄DMA | 2,2-Bis-[4-[oligo(ethoxy)]phenyl]propan-dimethacrylat (durchschnittlich 4 Ethoxyeinheiten pro Molekül) |
| HEMA | 2-Hydroxyethylmethacrylat |
| BTBACl | Benzyltributylammoniumchlorid |
| CuDMA | Kupferdimethacrylat |
| BHT | 2,6-Ditertiärbutyl-4-methylphenol |
| PBS | Phenylbenzylbarbitursäure |
| PK (Perketal) | 2,2-Di-(tert.-butylperoxy)-butan (ca. 50%ige Lsg. in Isododecan) |
| BPO (Diacylperoxid) | Dibenzoylperoxid |
| CU (Hydroperoxid) | Cumolhydroperoxid |
| AEC (Percarbonat) | Tert.-Amyl-peroxy-2-ethylhexylcarbonat |
| R812S | Aerosil® R812S (Degussa) |
| Bariumglas, MEMO-sil. | Bariumglas in d₅₀=1,5 µm Mahlung silanisiert mit 3,8 Gew.% 3-Methacryloylpropyltrimethoxysilan |
| Bariumglas, Vinyl-sil | Bariumglas in d₅₀=1,5 µm Mahlung silanisiert mit 3,0 Gew.% Triethoxyvinylsilan |
| PEG 400 | Polyethylenglycol, MW 400 |

### Prüfungsmethoden

Alle Prüfungen wurden, sofern nicht anders angegeben bei einer Temperatur von 23 ± 1°C durchgeführt.

### Biegefestigkeit/Biegemodul

Die Pastenkomponenten der zu vermessenden Pastenkombinationen werden luftfrei in die Kammern einer 10 ml Sicherheitsdoppelkartusche (Fa. Sulzer MixPac) mit einem Mischungsverhältnis der Komponenten von 10:1 eingefüllt. Gemäß ISO 4049 werden hieraus durch automatische Mischung (Mischkanüle MLX 3.2-12-S 10:1 grün der Firma Sulzer Mixpac) je Materialkombination 5-6 Prüfkörper hergestellt, gelagert und vermessen. Die Mittelwerte und Standardabweichungen sind jeweils in den Ergebnistabellen angegeben.

### Erhärtungszeit

Die Enderhärtungszeit (EHZ) der automatisch gemischten Pasten wurde im Rheometer mit koaxialen Zylindern ermittelt. Hierzu wurde jeweils eine etwa konstante Pastenmenge auf einen unteren Kunststoffzylinder gegeben. 30s nach Mischbeginn wurde ein permanent auf 37°C temperierter oberer Metallhohlzylinder bis zu einem definierten Abstand zum unteren Zylinder in die Paste gedrückt. Die beiden Zylinder wurden oszillierend gegeneinander bewegt und die hierfür erforderliche Kraft wurde mit Hilfe eines Schreibers aufgezeichnet. Die Enderhärtungszeit war diejenige Zeit, ab der die aufgezeichnete Kraft konstant blieb.

### Gelzeit

Das Material wird aus der Sicherheitsdoppelkartusche innerhalb weniger Sekunden automatisch gemischt und auf einen Mischblock appliziert. Die Paste wird anschließend im Abstand weniger Sekunden immer wieder umgewälzt. Wenn die Vergelung der Pastenmischung soweit fortgeschritten ist, dass eine erkennbare Elastizität eintritt und ein homogenes Durchmischen nicht mehr möglich ist, ist der Gelzeitpunkt erreicht. Angegeben ist die Zeit vom Ende des automatischen Mischvorgangs bis zum Gelzeitpunkt.

### Barcolhärte

Das Material wird aus der Sicherheitsdoppelkartusche mit Mischkanüle innerhalb weniger Sekunden in eine zylindrische Form aus nichtrostendem Stahl mit einer Höhe von 2,5 ± 0,1 mm und einem Durchmesser von 25 ± 0,1 mm gegeben und aushärten gelassen. Die so erhaltenen Prüfkörper wurden für ca. 24 h in Wasser bei 37°C gelagert. Die Prüfkörper wurden entnommen und die Barcolhärte mit Hilfe eines Barber-Colman-Impressors bestimmt. Dabei werden jeweils 5 Werte verteilt über einem Prüfkörper bestimmt und deren Mittelwert gebildet.

### Herstellungsbeispiel 1 (BTBACl-Lösung)

10 Gewichtsteile BTBACl werden mithilfe eines Magnetrührers in 90 Gewichtsteilen HEMA bis zur homogenen Lösung gerührt.

### Herstellungsbeispiel 2 (CuDMA-Lösung)

2 Gewichtsteile CuDMA werden mithilfe eines Magnetrührers in 98 Gewichtsteilen HEMA bis zur klaren Lösung gerührt.

### Beispiele und Referenzbeispiele (Vergleichsbeispiele)

In einem Glasbehälter werden zunächst die flüssigen Komponenten, die Lösungen aus den Herstellungsbeispielen sowie, sofern in der Rezeptur enthalten, Peroxide eingewogen, so dass die Anteile, wie in der nachfolgenden Tabellen angegeben, erreicht werden. Die so erstellten Mischungen werden bis zur klaren Lösung mithilfe eines Überkopfrührers gerührt.

Die jeweiligen Harzmischungen werden quantitativ in einen Labormischer überführt. R812S und silanisiertes Bariumglas werden bis zur homogenen Verteilung der Füllstoffe im Gemisch eingerührt. Die so hergestellten Pasten werden über einen Dreiwalzenstuhl (Fa. Exakt) gewalzt und dann unter Vakuum entgast. Die Pasten werden jeweils paarig innerhalb eines Beispiels wie in den Tabellen angegeben untersucht. Alle Mengenangaben erfolgen in Gewichtsteilen.

### Zusammensetzungen

### Peroxid in der zweiten Komponente (Weichmacherkomponente)

### Zweite Komponente (Weichmacherkomponente)

| | **Ref. 1** | **Ref. 2** | **Ref. 3** | **Ref. 4** | **Ref. 5** | **Bsp. 1** |
|---|---|---|---|---|---|---|
| | | | | | | |
| PEG 400 | 44,0 | 44,0 | 43,35 | 44,78 | 44,46 | 41,0 |
| Bariumglas Vinyl-Sil. | 50,0 | 50,0 | 49,2 | 49,2 | 49,2 | 50,0 |
| Aerosil R812-S | 3,0 | 3,0 | 2,9 | 2,9 | 2, 9 | 3,0 |
| PBS | 3,0 | - | 3,0 | 3,0 | 3,0 | 3,0 |
| PK | - | 3,0 | - | - | - | 3,0 |
| HX | - | - | - | - | - | - |
| BPO | - | - | 1,55 | -- | - | - |
| CU | - | - | - | 0,12 | - | - |
| AEC | - | - | - | - | 0,44 | - |

### Erste Komponente (Harzkomponente) für Ref. 1 - 5 und Bsp. 1

| | |
|---|---|
| Bis-GMA | 7,188 |
| UDMA | 13,92 |
| TEDMA | 2,085 |
| BPA(EO)₄DMA | 23,2 |
| HEMA | 0,548 |
| BHT | 0,007 |
| Cu-DMA | 0,002 |
| BTBACl | 0,05 |
| Bariumglas MEMO-Sil. | 50,0 |
| Aerosil R812-S | 3,0 |

### Peroxid in ersten Komponente (Harzkomponente)

### Beispiel 2

### Zweite Komponente (Weichmacherkomponente)

| | |
|---|---|
| PEG 400 | 44,0 |
| Bariumglas Vinyl-Sil. | 48,5 |
| Aerosil R812-S | 3,0 |
| PBS | 3,0 |
| BTBACl | 1,5 |

### Erste Komponente (Harzkomponente)

| | |
|---|---|
| Bis-GMA | 7,266 |
| UDMA | 14,07 |
| TEDMA | 2,107 |
| BPA(EO)₄DMA | 23,45 |
| HEMA | 0,098 |
| BHT | 0,007 |
| Cu-DMA | 0,002 |
| Bariumglas MEMO-Sil. | 49,0 |
| Aerosil R812-S | 3,0 |
| PK | 1,0 |

### Eigenschaften

| | **Ref. 1** | **Ref. 2** | **Ref. 3** | **Ref. 4** | **Ref. 5** | **Bsp. 1** | **Bsp. 2** |
|---|---|---|---|---|---|---|---|
| Gelzeit[s] Start 4 W. 40°C | 120 | Keine Aushärtung nach 40h | 69 | 37 | 74 | 90 | 112 |
| | 125 | | keine Aushärtung | 103 | 140 | 120 | 128 |
| EHZ [s] Start 4 W. 40°C | 570 | | 636 | 168 | 219 | 450 | 462 |
| | 294 | | keine Aushärtung | 297 | 348 | 282 | 378 |
| BF [MPa] Start 4 W. 40°C | 15 ± 1 | | 11 ± 1 | 75 ± 5 | 86 ± 7 | 50 ± 2 | 50 ± 4 |
| | 30 ± 2 | | keine Aushärtung | 38 ± 4 | 33 ± 3 | 53 ± 5 | 68±6 |
| BM [GPa] Start 4 W. 40°C | 0,39±0,04 | | 0,24±0,05 | 2,8 ± 0,1 | 3,2 ± 0,3 | 1,4 ± 0,1 | 1,4 ± 0,1 |
| | 1,0 ± 0,1 | | keine Aushärtung | 1,3 ± 0,1 | 1,1 ± 0,2 | 2,0 ± 0,3 | 2,4±0,2 |
| Barcolh. 24h Start 4 W. 40°C | 14 | | 14 | 54 | 55 | 44 | 42 |
| | 34 | | keine Aushärtung | 41 | 41 | 53 | 52 |

Es zeigt sich, dass die Beispiele 1 und 2, die Barbitursäure und das erfindungsgemäße Perketal enthalten, auch nach einer Lagerzeit von 4 Wochen bei 40°C stabile und gegenüber dem Peroxidfreien System (Ref. 1) signifikant verbesserte mechanische Eigenschaften aufweisen.

Die anfänglich bei Verwendung des Percarbonats (AEC, Ref. 5, EP 1 194 110 Soglowek) gefundenen deutlich verbesserten mechanischen Eigenschaften verschlechtern sich nach Lagerung der Komponenten vor deren Verarbeitung deutlich, obwohl das Material nach wie vor eine befriedigende Erhärtung nach Messung der Erhärtungszeit zeigt. Offensichtlich zeigen Zusammensetzungen dieses Typs keine ausreichenden Stabilitäten in Bezug auf diese mechanischen Eigenschaften. Dies ist in Anbetracht der recht hohen thermischen Stabilitäten des AEC (T_{1/2}(10h) = 95°C) kein vorhersehbarer Effekt (z.Vgl. PK: T_{1/2}(10h) - 98°C; beide Peroxide haben also einen ähnlich schnellen thermischen Zerfall).

Ähnlich wie bei dem Percarbonat ist die Mechanik des ausgehärteten Materials beim Hydroperoxid (CU, Ref. 4) über die Lagerzeit der Pasten nicht stabil und reduziert sich nach 4 Wochen bei 40°C deutlich.

Das Diacylperoxid (BPO, Ref. 3) zeigt keinerlei positive Wirkung für die mechanischen Eigenschaften des erhärteten Materials und deaktiviert darüber hinaus vollständig bei Lagerung der unausgehärteten Pasten.

Ref. 2 zeigt schließlich, dass die Initiatorsysteme auf die Anwesenheit der CH-aciden Verbindung (PBS, Barbitursäure) angewiesen sind, da es andernfalls nicht zur Erhärtung kommt.

## Patentansprüche

1. Zwei- oder Mehrkomponentensystem zur Herstellung eines Dentalmaterials, das enthält:
a) eine erste Komponente, die enthält:
i. wenigstens ein radikalisch polymerisierbares Harz,
ii. wenigstens einen Polymerisationsbeschleuniger,
b) eine zweite Komponente, die enthält:
i. eine bei Raumtemperatur flüssige oder pastöse inerte Matrix und/oder wenigstens ein radikalisch polymerisierbares Harz,
ii. wenigstens eine CH-acide Verbindung und/oder ein Salz einer CH-aciden Verbindung als Polymerisationsinitiator,
wobei die zweite Komponente nur dann wenigstens ein radikalisch polymerisierbares Harz enthält, wenn der Polymerisationsinitiator nach ii. ein Salz einer CH-aciden Verbindung ist,
**dadurch gekennzeichnet, dass** die erste und/oder zweite Komponente mindestens ein Perketal enthält.

2. Zwei- oder Mehrkomponentensystem nach Anspruch 1, **dadurch gekennzeichnet, dass** es 0,001 bis 5 Gew.-%, weiter vorzugsweise 0,01 bis 3 Gew.-%, weiter vorzugsweise 0,02 bis 2 Gew.-%, weiter vorzugsweise 0,02 bis 1,5 Gew.-%, weiter vorzugsweise 0,03 bis 1,0 Gew.-% des Perketals enthält.

3. Zwei- oder Mehrkomponentensystem nach Anspruch 1 oder 2, **dadurch gekennzeichnet, dass** das Perketal in der zweiten Komponente enthalten ist.

4. Zwei- oder Mehrkomponentensystem nach einem der Ansprüche 1 bis 3, **dadurch gekennzeichnet, dass** die 10 h Halbwertstemperatur (T_{½}(10h)) des Perketals mindestens 75°C, vorzugsweise mindestens 80°C beträgt.

5. Zwei- oder Mehrkomponentensystem nach einem der Ansprüche 1 bis 4, **dadurch gekennzeichnet, dass** die CH-acide Verbindung ausgewählt ist aus der Gruppe bestehend aus α-Benzoylpropionitrilen, α-Cyancarbonsäureestern und -amiden, cyclischen β-Oxonitrilen, β-Diketonen, cyclischen β-Diketonen, cyclischen β-Oxocarbonsäureestern, cyclischen β-Oxolactonen, Malonsäuren, insbesondere Malonylsulfamid, Pyrazolen, insbesondere Pyrazolon und Pyrazolidin, Barbitursäuren, Barbitursäurederivaten, Thiobarbitursäuren, Thiobarbitursäurederivaten sowie Salzen, insbesondere Erdalkali- und Alkalisalzen sowie teilweise oder vollständig alkylierten oder arylierten oder gemischt alkylierten oder arylierten Ammonium- oder Phosphoniumsalzen vorgenannter Verbindungen.

6. Zwei- oder Mehrkomponentensystem nach einem der Ansprüche 1 bis 5, **dadurch gekennzeichnet, dass** der Polymerisationsbeschleuniger im Harz lösliche Metallverbindungen umfasst, die zum Wechsel der Oxidationsstufe befähigte Übergangsmetallionen enthalten.

7. Zwei- oder Mehrkomponentensystem nach einem der Ansprüche 1 bis 6, **dadurch gekennzeichnet, dass** es zusätzlich ionogene Verbindungen, bevorzugt ionogene Halogenid- und/oder Pseudohalogenidverbindungen enthält, wobei diese Verbindungen bevorzugt in der ersten Komponente enthalten sind.

8. Zwei- oder Mehrkomponentensystem nach einem der Ansprüche 1 bis 7, **dadurch gekennzeichnet, dass** es zusätzlich ein Fotoinitiatorsystem enthält.

9. Zwei- oder Mehrkomponentensystem nach einem der Ansprüche 1 bis 8, **dadurch gekennzeichnet, dass** das radikalisch polymerisierbare Harz (Meth)acrylate, Di(meth)acrylate und/oder höher funktionalisierte (Meth)acrylate umfasst.

10. Zwei- oder Mehrkomponentensystem nach einem der Ansprüche 1 bis 9, **dadurch gekennzeichnet, dass** die bei Raumtemperatur flüssige oder pastöse inerte Matrix Weichmacher enthält, bevorzugt Polethylenglycolderivate wie Polyethylenoxid, 2,2-Bis-[4-[oligo(ethoxy)]phenyl]propan-diacetat, 2,2-Bis-[4-[2-hydroxyethoxy]phenyl]propan-diacetat; Polypropylenglycolderivate; andere Poly- und Oligoether; Polyester; Phthalsäureester; Silikonöle; oder Paraffinöle.

11. Zwei- oder Mehrkomponentensystem nach einem der Ansprüche 1 bis 10, **dadurch gekennzeichnet, dass** es zusätzlich dentalübliche Additive ausgewählt aus der Gruppe bestehend aus Füllstoffen, Thixotropiermitteln, Pigmenten, Trocknungsmitteln, Fluoreszenzfarbstoffen, antibakteriellen Wirkstoffen, Fluoriden und Stabilisatoren enthält.

12. Verwendung eines Zwei- oder Mehrkomponentensystems nach einem der Ansprüche 1 bis 11 zur Herstellung eines Dentalmaterials.

13. Verwendung nach Anspruch 12, **dadurch gekennzeichnet, dass** das Dentalmaterial ausgewählt ist aus der Gruppe bestehend aus Füllungsmaterialien, Adhäsiven, Stumpfaufbaumaterialien, Kronen- und Brückenmaterialien, provisorischen Kronen- und Brückenmaterialien, sowie Zementen.

14. Kit zur Herstellung eines Dentalmaterials, enthaltend ein Zwei- oder Mehrkomponentensystem nach einem der Ansprüche 1 bis 11 in Behältern.

15. Kit nach Anspruch 14, **dadurch gekennzeichnet, dass** er zusätzlich eine Mischvorrichtung aufweist.

## Claims

1. A two-component or multicomponent system for producing a dental material, comprising:
a) a first component, comprising:
i. at least one radically polymerizable resin,
ii. at least one polymerization accelerator;
b) a second component, comprising:
i. an inert matrix which is pasty or liquid at room temperature, and/or at least one radically polymerizable resin,
ii. at least one CH-acidic compound and/or a salt of a CH-acidic compound, as polymerization initiator,
where the second component comprises at least one radically polymerizable resin only when the polymerization initiator according to ii. is a salt of a CH-acidic compound,
**characterized in that** the first and/or second component comprises at least one perketal.

2. The two-component or multicomponent system of claim 1, **characterized in that** it comprises 0.001% to 5% by weight, more preferably 0.01% to 3% by weight, more preferably 0.02% to 2% by weight, more preferably 0.02% to 1.5% by weight, more preferably 0.03% to 1.0% by weight of the perketal.

3. The two-component or multicomponent system of claim 1 or 2, **characterized in that** the perketal is present in the second component.

4. The two-component or multicomponent system of any of claims 1 to 3, **characterized in that** the 10 h half-life temperature (T_{1/2}(10 h)) of the perketal is at least 75°C, preferably at least 80°C.

5. The two-component or multicomponent system of any of claims 1 to 4, **characterized in that** the CH-acidic compound is selected from the group consisting of α-benzoylpropionitriles, α-cyanocarboxylic esters and amides, cyclic β-oxonitriles, β-diketones, cyclic β-diketones, cyclic β-oxocarboxylic esters, cyclic β-oxolactones, malonic acids, more particularly malonyl-sulfamide, pyrazoles, more particularly pyrazolone and pyrazolidine, barbituric acids, barbituric acid derivatives, thiobarbituric acids, thiobarbituric acid derivatives, and also salts, more particularly alkaline earth metal salts and alkali metal salts, and also partly or fully alkylated or arylated or mixedly alkylated or arylated ammonium or phosphonium salts of aforesaid compounds.

6. The two-component or multicomponent system of any of claims 1 to 5, **characterized in that** the polymerization accelerator comprises metal compounds which are soluble in the resin and which comprise transition metal ions capable of a change in oxidation state.

7. The two-component or multicomponent system of any of claims 1 to 6, **characterized in that** it further comprises ionic compounds, preferably ionic halide and/or pseudohalide compounds, these compounds being present preferably in the first component.

8. The two-component or multicomponent system of any of claims 1 to 7, **characterized in that** it further comprises a photoinitiator system.

9. The two-component or multicomponent system of any of claims 1 to 8, **characterized in that** the radically polymerizable resin comprises (meth)acrylates, di(meth)acrylates and/or more highly functionalalized (meth)acrylates.

10. The two-component or multicomponent system of any of claims 1 to 9, **characterized in that** the inert matrix which is pasty or liquid at room temperature comprises plasticizers, preferably polyethylene glycol derivatives such as polyethylene oxide, 2,2-bis[4-[oligo(ethoxy)]phenyl]propane diacetate, 2,2-bis[4-[2-**hydroxyethoxy**]phenyl]propane diacetate; polypropylene glycol derivatives; other polyethers and oligoethers; polyesters; phthalic esters; silicone oils; or liquid paraffins.

11. The two-component or multicomponent system of any of claims 1 to 10, **characterized in that** it further comprises customary dental additives selected from the group consisting of fillers, thixotropic agents, pigments, dryers, fluorescent dyes, active antibacterial ingredients, fluorides, and stabilizers.

12. The use of a two-component or multicomponent system of any of claims 1 to 11 for producing a dental material.

13. The use of claim 12, **characterized in that** the dental material is selected from the group consisting of filling materials, adhesives, core build-up materials, crown and bridge materials, temporary crown and bridge materials, and cements.

14. A kit for producing a dental material, comprising a two-component or multicomponent system of any of claims 1 to 11 in containers.

15. The kit of claim 14, **characterized in that** it additionally includes a mixing means.

## Revendications

1. Système à deux ou plusieurs composants pour la préparation d'un matériau dentaire, qui contient :
a) un premier composant, qui contient :
i. au moins une résine polymérisable radicalairement,
ii. au moins un accélérateur de polymérisation,
b) un deuxième composant, qui contient :
i. une matrice inerte liquide ou pâteuse à la température ambiante et/ou au moins une résine polymérisable radicalairement,
ii. au moins un composé avec un CH acide et/ou au moins un sel d'un composé avec un CH acide comme initiateur de polymérisation,
sachant que le deuxième composant ne contient au moins une résine polymérisable radicalairement que quand l'initiateur de polymérisation selon ii. est un sel d'un composé avec un CH acide,
**caractérisé en ce que** le premier et/ou le deuxième composant contiennent au moins un percétal.

2. Système à deux ou plusieurs composants selon la revendication 1, **caractérisé en ce qu'**il contient de 0,001 à 5 % en poids, de préférence de 0,01 à 3 % en poids, plus préférablement de 0,02 à 2 % en poids, encore plus préférablement de 0,02 à 1,5 % en poids, de façon particulièrement préférée de 0,03 à 1,0 % en poids du percétal.

3. Système à deux ou plusieurs composants selon la revendication 1 ou 2, **caractérisé en ce que** le percétal est contenu dans le deuxième composant.

4. Système à deux ou plusieurs composants selon l'une quelconque des revendications 1 à 3, **caractérisé en ce que** la température pour une demi-vie au bout de 10 h (T_{1/2}(10h)) du percétal est d'au moins 75 °C, de préférence d'u moins 80 °C.

5. Système à deux ou plusieurs composants selon l'une quelconque des revendications 1 à 4, **caractérisé en ce que** le composé à CH acide est choisi parmi le groupe consistant en les α-benzoylpropionitriles, les esters et amides d'acides α-cyanocarboxyliques, les β-oxonitriles cycliques, les β-dicétones, les β-dicétones cycliques, les esters cycliques d'acides β-oxocarboxyliques, les β-oxolactones cycliques, les acides maloniques, en particulier le malonylsulfamide, les pyrazoles, en particulier la pyrazolone et la pyrazolidine, les acides barbituriques, les dérivés d'acides barbituriques, les acides thiobarbituriques, les dérivés d'acides thiobarbituriques, ainsi qu'en des sels des composés susmentionnés, en particulier des sels alcalino-terreux et alcalins, ainsi que des sels d'ammonium ou de phosphonium partiellement ou complètement alkylés ou arylés, ou alkylés ou arylés mixtes.

6. Système à deux ou plusieurs composants selon l'une quelconque des revendications 1 à 5, **caractérisé en ce que** l'accélérateur de polymérisation comprend des composés métalliques solubles dans la résine, qui contiennent des ions de métaux de transition aptes au changement de leur degré d'oxydation.

7. Système à deux ou plusieurs composants selon l'une quelconque des revendications 1 à 6, **caractérisé en ce qu'**il contient en plus des composés ionogènes, de préférence des composés ionogènes d'halogénures et/ou de pseudo-halogénures, ces composés étant contenus de préférence dans le premier composant.

8. Système à deux ou plusieurs composants selon l'une quelconque des revendications 1 à 7, **caractérisé en ce qu'**il contient en plus un système de photoinitiateurs.

9. Système à deux ou plusieurs composants selon l'une quelconque des revendications 1 à 8, **caractérisé en ce que** la résine polymérisable radicalairement comprend des (méth)acrylates, des di(méth)acrylates et/ou des (méth)acrylates de fonctionnalité supérieure.

10. Système à deux ou plusieurs composants selon l'une quelconque des revendications 1 à 9, **caractérisé en ce que** la matrice inerte, liquide ou pâteuse à la température ambiante, contient des plastifiants, de préférence des dérivés du polyéthylène glycol comme le poly(oxyéthylène), le diacétate de 2,2-bis-[4-[oligo(éthoxy)phényl]propane, le diacétate de 2,2-bis-[4-(2-hydroxyéthoxy)phényl]propane, des dérivés du polypropylène glycol, d'autres poly- et oligoéthers, des polyesters, des esters phtaliques, des huiles de silicones ou des huiles de paraffines.

11. Système à deux ou plusieurs composants selon l'une quelconque des revendications 1 à 10, **caractérisé en ce qu'**il contient en plus des additifs dentaires habituels, choisis parmi le groupe consistant en des charges, des agents thixotropiants, des pigments, des siccatifs, des colorants fluorescents, des principes actifs antibactériens, des fluorures et des stabilisateurs.

12. Utilisation d'un système à deux ou plusieurs composants selon l'une quelconque des revendications 1 à 11 à la préparation d'un matériau dentaire.

13. Utilisation selon la revendication 12, **caractérisé en ce que** le matériau dentaire est choisi parmi le groupe consistant en les matériaux d'obturation, les adhésifs, les matériaux de construction de moignons, les matériaux de couronne et de bridge, ainsi que les ciments.

14. Kit pour la préparation d'un matériau dentaire, contenant un système à deux ou plusieurs composants selon l'une quelconque des revendications 1 à 11 dans des récipients.

15. Kit selon la revendication 14, **caractérisé en ce qu'**il comporte en plus un dispositif de mélangeage.
